(19) Europäisches Patentamt
European Patent Office
Office européen des brevets

(11) Veröffentlichungsnummer: **0 087 655**
**B1**

(12) **EUROPÄISCHE PATENTSCHRIFT**

(45) Veröffentlichungstag der Patentschrift: **16.07.86**

(51) Int. Cl.[4]: **C 07 D 209/28**, C 07 D 405/12, C 07 D 413/12, A 61 K 31/405

(21) Anmeldenummer: **83101352.9**

(22) Anmeldetag: **12.02.83**

(54) **Verfahren zur Herstellung von Acemetacin.**

(30) Priorität: **26.02.82 DE 3206885**

(43) Veröffentlichungstag der Anmeldung: **07.09.83 Patentblatt 83/36**

(45) Bekanntmachung des Hinweises auf die Patenterteilung: **16.07.86 Patentblatt 86/29**

(84) Benannte Vertragsstaaten: **AT BE CH DE FR GB IT LI LU NL SE**

(56) Entgegenhaltungen:
**EP - A - 0 084 127**
**DE - A - 2 943 125**

**CHEMICAL ABSTRACTS, Band 87, 1977, Seite 500, Nr. 39279u, Columbus, Ohio, USA**
**"Protective groups in organic synthesis", 1981, Seiten 160,166,168,177**

(73) Patentinhaber: **Troponwerke GmbH & Co. KG, Berliner Strasse 156, D-5000 Köln 80 (DE)**

(72) Erfinder: **Boltze, Karl-Heinz, Dr., Ackerstrasse 8, D-5231 Borod (DE)**
Erfinder: **Horstmann, Harald, Dr., Claudiusweg 19, D-5600 Wuppertal 1 (DE)**

(74) Vertreter: **Jesse, Ralf-Rüdiger, Dr. et al, Bayer AG Konzernverwaltung RP Patentabteilung, D-5090 Leverkusen 1 Bayerwerk (DE)**

EP 0 087 655 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Acemetacin (1-(p-Chlorphenyl)-5-methoxy-2-methylindol-3-acetoxy-essigsäure), neue Indolderivate als Zwischenprodukte und ein Verfahren zu ihrer Herstellung.

Es ist bereits bekannt, Indolderivate als Antiphlogistika in der Human- und Veterinärmedizin einzusetzen. Beispielhaft seien genannt: das Cinmetacin [INN; 1-Cinnamoyl-5-methoxy-2-methyl-3-indolessigsäure] und Indometacin [INN; 1-(4-Chlorbenzoyl)-5-methoxy-2-methyl-3-indolessigsäure]. Beide Verbindungen haben eine starke Säurefunktion, so dass Nebenwirkungen unausbleiblich sind. So hat J. Solinca (vgl. Arzneimittelforschung 21, Nr. 11a (1971), Seite 1834) als leichtere Nebenwirkungen neurosensible Störungen wie Kopfschmerzen, Schwindel und Konzentrationsschwierigkeiten sehr häufig beobachtet, daneben frequente Verdauungsbeschwerden mit Appetitlosigkeit, Übelkeit, Magenschmerzen, Diarrhoe und schliesslich einige schwere Fälle von Darmblutungen, Magengeschwüren und zeitweise neurologische Ausfallerscheinungen festgestellt. G. Morandi und U. Serni (vgl. Arzneimittelforschung 21, Nr. 11a (1971), Seite 1834) stellten fest, dass bei nahezu jedem zehnten Patienten die Therapie wegen der Nebenwirkungen abgebrochen werden musste.

In der DE 2 234 651 und DE 2 943 115 werden Verfahren zur Herstellung von Acemetacin über den entsprechenden Benzylester als Zwischenprodukt beschrieben.

Nach CA 87, 39279u werden Methylenester der 1-(p-Chlorbenzyl)-5-methoxy-2-methylindol-3-essigsäure in Gegenwart von Kaliumjodid als Katalysator hergestellt.

Aus der älteren, aber nicht vorpublizierten EP 0 084 127 ist die Umsetzung von Indometacin über den entsprechenden Tetrahydropyranylester zu Acemetacin bekannt.

Es wurde ein Verfahren zur Herstellung von Acemetacin der Formel

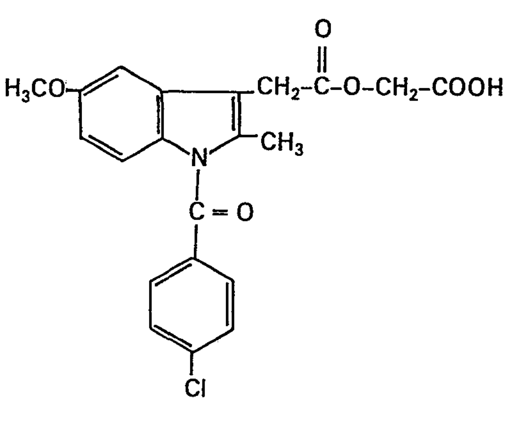

(I)

durch Umsetzung von Salzen der Indolcarbonsäure der Formel

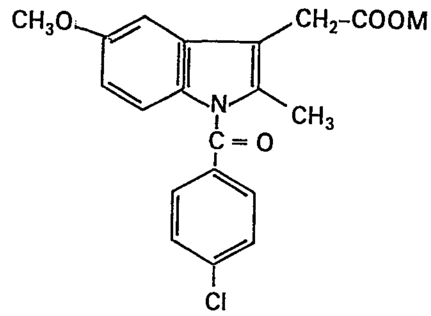

(II)

in der

M für Alkali oder der entsprechenden stöchiometrischen Menge Erdalkali steht,

mit Verbindungen der Formel

$Hal-CH_2-X-R$ (III),

in der

Hal für Chlor, Brom oder Jod steht,

X für -COO- oder -CONH- und

R für eine Tetrahydrofuran-2-yl, eine Alkoxybenzyl- oder eine geradkettige oder verzweigte Alkylgruppe steht, die gegebenenfalls durch eine Hydroxylgruppe substituiert sein kann oder wobei X-R gemeinsam einen Oxazolinring bilden, der durch Alkylgruppen substituiert sein kann,

in Gegenwart von inerten organischen Lösungsmitteln oder Gemischen von organischen Lösungsmitteln mit Wasser zu Indolderivaten der Formel

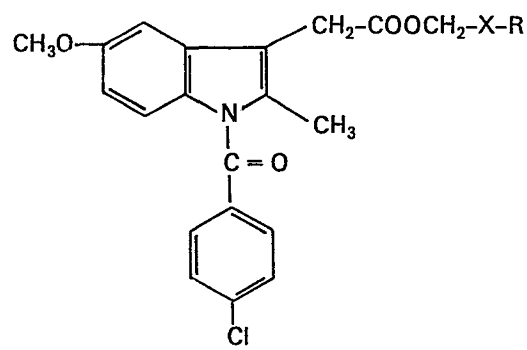

(IV)

in der

X und R die obengenannte Bedeutung haben,

und nachfolgender Hydrolyse gefunden, das dadurch gekennzeichnet ist, dass man die Hydrolyse in Gegenwart von Eisessig/Chlorwasserstoff oder p-Toluolsulfonsäure ausführt.

Nach dem erfindungsgemässen Verfahren erhält man überraschenderweise Acemetacin, welches frei von Deschlorverbindungen ist.

Es wurden ausserdem neue Indolderivate der Formel

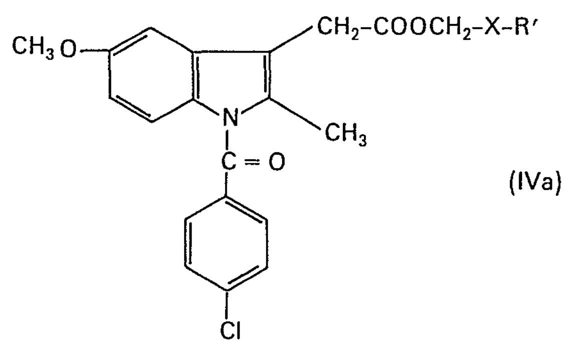

(IVa)

in der
X für -COO- oder -COHN- und
R′ für eine Tetrahydrofuran-2-yl, eine Alkoxybenzyl- oder eine geradkettige oder verzweigte Alkylgruppe steht, die gegebenenfalls durch eine Hydroxylgruppe substituiert sein kann, oder wobei X-R′ gemeinsam einen Oxazolinring bilden, der durch Alkylgruppen substituiert sein kann, sowie ihre mit Basen gebildeten pharmazeutisch unbedenklichen Salze, gefunden.

Die neuen Indolderivate sind Zwischenprodukte bei der Herstellung von Acemetacin.

Die neuen Indolderivate besitzen vorteilhafte Wirkungen bei entzündungsbedingten Erkrankungen sowie bei Erkrankungen des rheumatischen Formenkreises.

Die neuen Indolverbindungen lassen sich vorteilhaft als Ausgangsverbindungen zur Herstellung von Acemetacin, welches frei von Deschloracemetacin ist, einsetzen. Dies ist eine erfindungsgemäss bevorzugte Verwendung. Bei dieser Verwendung lassen sich gegebenenfalls unter Einsatz von Spaltungsreagentien die Schutzgruppen leicht abspalten und Acemetacin in hoher Ausbeute und Reinheit erhalten.

Gegenstand der Erfindung ist ausserdem ein Verfahren zur Herstellung der neuen Indolderivate, das dadurch gekennzeichnet ist, dass man Salze der Indolcarbonsäure der Formel

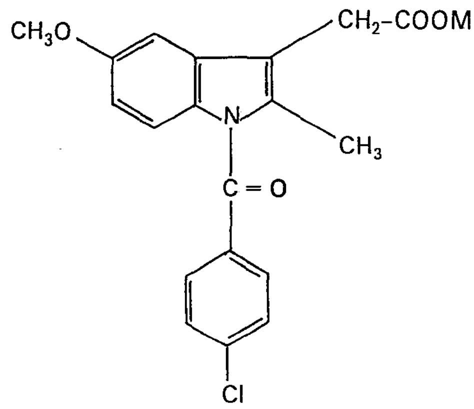

in der
M für Alkali- oder entsprechende stöchiometrische Mengen von Erdalkalimetallen steht,
mit Verbindungen der Formel

$Hal-CH_2-X-R'$

in der
Hal für Chlor, Brom, Jod, vorzugsweise Brom, steht und
X und R′ die oben genannte Bedeutung haben, in Gegenwart von inerten organischen Lösungsmitteln oder Gemischen von organischen Lösungsmitteln mit Wasser im allgemeinen in einem Temperaturbereich von −30 bis +70 °C umsetzt.

Das erfindungsgemässe Verfahren kann durch das folgende Formelschema wiedergegeben werden:

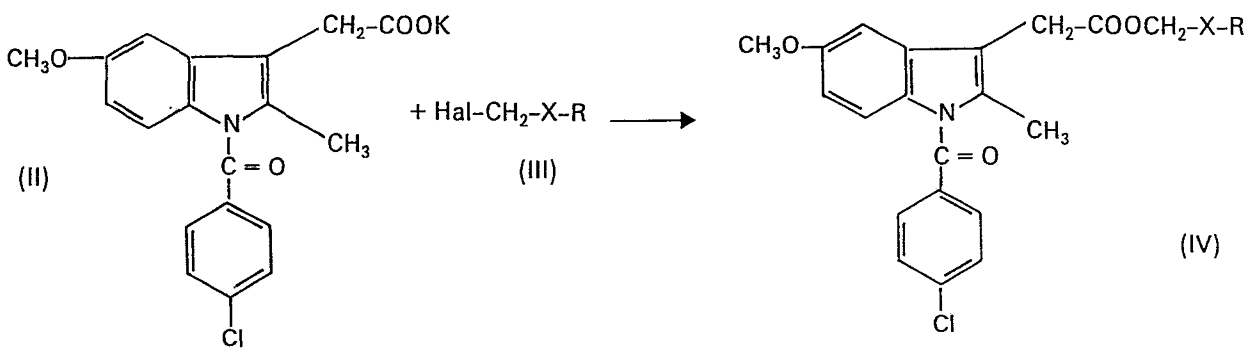


(II) (III) (IV)

Die als Ausgangsstoffe verwendeten Verbindungen der Formel (II) und (III) sind bekannt oder werden nach bekannten Verfahren hergestellt. Vorzugsweise werden als Verbindungen der Formel (III) die folgenden Stoffe eingesetzt:

Tetrahydrofuran-2-yl-2-bromacetat,
Tetrahydrofuran-2-yl-2-bromacetat,
4-Methoxybenzyl-2-bromacetat,
N-(1-Hydroxy-2-methylpropyl-2)-2-bromacetamid und
4.4-Dimethyl-2-oxazolin-2-yl-methylbromid.

Die Reaktion wird zweckmässigerweise in Gegenwart von Verdünnungsmitteln durchgeführt. Als Verdünnungsmittel kommen generell alle inerten Lösungsmittel, vorzugsweise inerte organische Lösungsmittel, in Frage. Hierzu gehören wiederum bevorzugt polare und aprotische Lösungsmittel wie z.B. Chloroform, Dichlormethan

und Dioxan. Besonders bevorzugt werden Tetrahydrofuran, Dimethylformamid und Hexamethylphosphorsäuretriamid eingesetzt.

Die Temperaturen können in einem grösseren Bereich variiert werden, im allgemeinen wird bei Temperaturen zwischen ca. −30 und +70 °C gearbeitet. Vorzugsweise wird zwischen 0 und 40 °C gearbeitet, besonders bevorzugt um Raumtemperatur, d.h. zwischen ca. 15 °C und 25 °C.

Die Umsetzung erfolgt bevorzugt unter Normaldruck.

Bei der Durchführung des erfindungsgemässen Verfahrens setzt man die Reaktionspartner II) und III) vorteilhafterweise in molaren Mengenverhältnissen ein. Die Aufarbeitung erfolgt allgemein durch Verdünnen der Reaktionslösung mit einem geeigneten, nicht mit Wasser mischbaren Lösungsmittel, Auswaschen der in Wasser löslichen Teile, Filtrieren der organischen Phase und Chromatographieren an $SiO_2$. Die neuen erfindungsgemässen Indolderivate, die auch als Acemetacinderivate bezeichnet werden können, können bei Bedarf in leichter Weise mittels Spaltungsreagentien in Acemetacin übergeführt werden.

Von besonderer Bedeutung sind die nachstehenden Verbindungen, von denen die Pyranylesterverbindung noch bevorzugt wird:

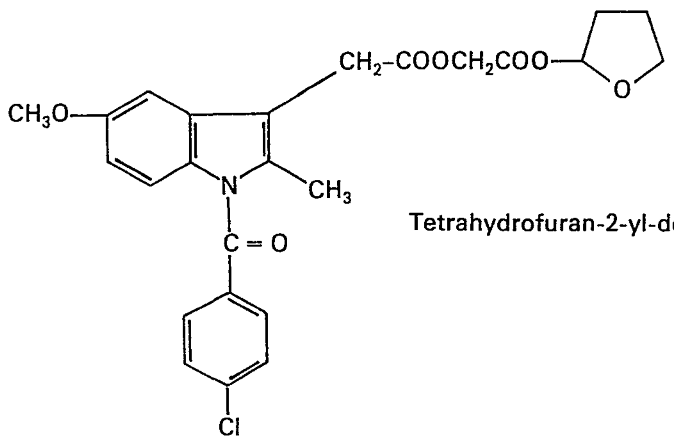

Tetrahydrofuran-2-yl-derivat

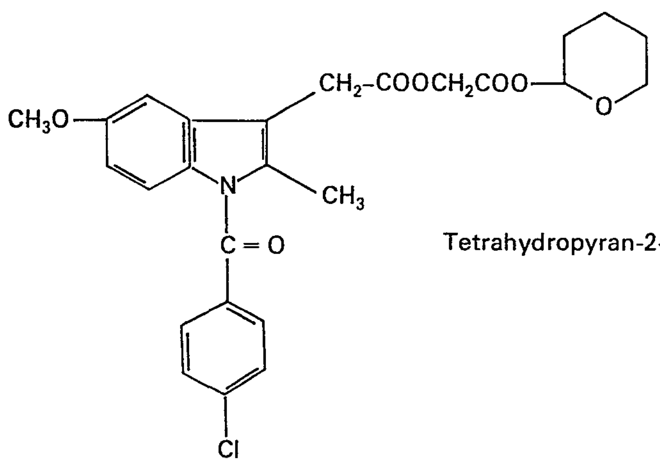

Tetrahydropyran-2-yl-derivat

Die neuen Wirkstoffe weisen starke entzündungshemmende Wirkungen auf. So wurden an dem bekannten pharmakologischen Entzündungsmodell, dem Kaolinödem der Rattenpfote (vgl. Kemper, Z.ges.exp.Med. 131, 407 (1959)) Ergebnisse erzielt, die denen der in der Pharmazie verwendeten Entzündungshemmer entsprechen und sie teilweise sogar übertreffen. Auch in der Steigerung der Sulfhydrylgruppenaktivität von Serumproteinen, welche ein Mass für die Wirksamkeit von Antiphlogistika darstellt (vgl. D.A. Gerber et al., Biochem. Pharmacol. 16, 115 (1967)), waren die Mehrzahl der erfindungsgemässen Verbindungen der als Vergleichssubstanz verwendeten Flufenaminsäure deutlich überlegen. Die erfindungsgemässen Verbindungen versprechen daher bei der Bekämpfung von Erkrankungen des rheumatischen Formenkreises eine Bereicherung der Pharmazie zu werden.

Im allgemeinen hat es sich sowohl in der Human- als auch in der Veterinärmedizin als vorteilhaft erwiesen, den oder die erfindungsgemässen Wirkstoffe in Gesamtmengen von etwa 0,1 bis etwa 200, vorzugsweise 0,5 bis 10 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemässen Wirkstoffe, vorzugsweise in Mengen von etwa 0,1 bis etwa 70, insbesondere 0,1 bis 2,0 mg/kg Körpergewicht. Es kann jedoch erforderlich sein, von den genannten Dosierungen abzuweichen und zwar in Abhängigkeit von der Art und dem Körpergewicht des zu behandelnden Objektes, der Art und der Schwere der Erkrankung.

Die neuen Acemetacinderivate werden in jeweils entsprechendem Beispiel aufgrund ihres chemischen Namens und ihrer Formel charakterisiert. In den Beispielen ist entsprechend der allgemeinen Formel (I) nur der jeweils für -X-R stehende Rest angegeben.

Beispiele

Beispiel 1

Herstellung von Tetrahydrofuran-2-yl-[1-(4-Chlorbenzoyl)-5-methoxy-2-methylindol-3-acetoxy]-acetat

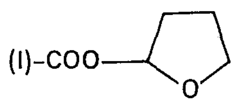

Tetrahydrofuran-2-yl-2-bromacetat

Das Glykolsäurederivat wird durch Reaktion von 6,95 g (0,05 Mol) Bromessigsäure und 2,80 g (0,4 Mol) 2,3-Dihydrofuran bei Raumtemperatur unter Feuchtigkeitsabschluss und Rühren nach 5 h (Bromessigsäure ist quantitativ umgesetzt) erhalten. Diese Reaktionslösung wurde ohne eine Isolierung im folgenden Syntheseschritt eingesetzt. Das Glykolsäurederivat hat die Formel:

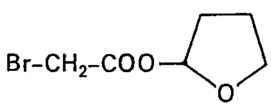

Acemetacinderivat

3,58 g (0,01 Mol) 1-(4-Chlorbenzoyl)-5-methoxy-2-methylindol-3-essigsäure löst man in 20 ml abs. DMF, setzt 0,7 g (0,0051 Mol) getrocknetes und pulverisiertes Kaliumcarbonat zu und lässt unter Rühren bei 55 °C die Bildung des Kaliumsalzes ablaufen.

In die klare Lösung des Kaliumsalzes tropft man anschliessend bei Raumtemperatur 7 g obiger Reaktionslösung = 2,1 g (0,01 Mol). Nach 2 h ist die Umsetzung beendet (pH ca. 4).

Das Reaktionsgemisch wird auf 0 °C gekühlt, mit 200 ml kaltem Essigsäureethylester versetzt und nacheinander mit eiskalter, halbkonzentrierter und eiskalter stärker verdünnter Kaliumbicarbonatlösung sowie 3mal mit Eiswasser ausgeschüttelt. Nach Trocknung über $Na_2SO_4/K_2CO_3$ entfernt man am Rotationsverdampfer bei 10 °C und 40 Torr den Überschuss an 2,3-Dihydrofuran und das Lösungsmittel und erhält als Rückstand 6,8 g gelbes Öl.

Die Kristallisation des Öles setzt unter Feuchtigkeitsabschluss (Natronkalkrohr) in der Tiefkühltruhe nach 2 Tagen ein. Zur Vervollständigung der Kristallisation werden 4 ml Tetrachlorkohlenstoff (über $K_2CO_3$ getrocknet) gut eingerührt und die Mischung in der Kälte (Kühltruhe) aufbewahrt.

Das abgesaugte Kristallisat wird mit wenig $CCl_4$ gewaschen und im Exsikkator über KOH/Paraffinschnitzel getrocknet. Ausbeute: 69,6% der Theorie; Fp.: 86–88 °C.

Herstellung von 1-(4-Chlorbenzoyl)-5-methoxy-2-methyl-indol-3-acetoxyessigsäure-Acemetacin

Abspaltung der Schutzgruppe des erfindungsgemässen Acemetacinderivates

Der im vorstehenden Versuch hergestellte Tetrahydrofuranylester (3,3 g) wird in 30 ml Eisessig gelöst und 30 Minuten auf 40 bis 50 °C erwärmt. Hierbei erfolgte die Abspaltung der Schutzgruppe quantitativ.

Nach Abdestillieren des Eisessigs wird der Rückstand aus Tetrachlorkohlenstoff kristallisiert. Das abgesaugte Kristallisat trocknet man im Exsikkator über KOH.

Die vom Lösungsmittel befreite Mutterlauge wird mit 10 ml halbkonzentrierter Kaliumbicarbonatlösung und 15 ml Tetrahydrofuran versetzt, gut durchgerührt und mit 3mal 20 ml Ether extrahiert. Die alkalische Phase säuert man an, extrahiert mit Essigsäureethylester und wäscht die organische Phase neutral. Nach Trocknung über $Na_2SO_4$ engt man ein und kristallisiert den Rückstand aus Tetrachlorkohlenstoff.

Gesamtausbeute: 3,5 g = 84,2% der Theorie.

Beispiel 2

Herstellung von Tetrahydropyran-2-yl-[1-(4-Chlorbenzoyl)-5-methoxy-2-methylindol-3-acetoxy]-acetat

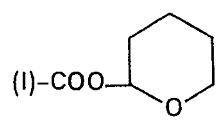

Tetrahydropyran-2-yl-2-bromacetat

Das Glykolsäurederivat wird durch Reaktion von 6,95 g (0,05 Mol) Bromessigsäure und 33,6 g (0,4 Mol) 3,4-Dihydro-2 H-pyran bei Raumtemperatur unter Feuchtigkeitsausschluss, $N_2$-Schutz und Rühren nach 17 h (quantitativer Umsetzung von Bromessigsäure nach DC und NMR) erhalten. Diese Reaktionslösung setzt man ohne Isolierung im folgenden Syntheseschritt ein. Das Glykolsäurederivat hat die Formel:

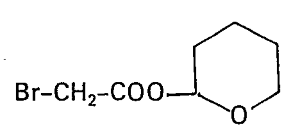

Acemetacinderivat

3,58 g (0,01 Mol) 1-(4-Chlorbenzoyl)-5-methoxy-2-methyl-indol-3-essigsäure löst man in 20 ml abs. DMF, setzt 0,7 g (0,0051 Mol) getrocknetes und pulverisiertes Kaliumcarbonat zu und lässt

unter Rühren 6 h bei 55 °C die Bildung des Kaliumsalzes ablaufen.

In die klare Lösung des Kaliumsalzes tropft man anschliessend bei Raumtemperatur innerhalb von 10 Minuten 8,1 g obiger Reaktionslösung (ca. 0,01 Mol). Nach 2 h ist die Umsetzung beendet (pH 6).

Das Reaktionsgemisch wird mit 100 ml Essigsäureethylester versetzt und nacheinander mit halbkonzentrierter und 4mal mit stärker verdünnter Kaliumbicarbonatlösung ausgeschüttelt. Nach Trocknung über $Na_2SO_4/K_2CO_3$ filtriert man und entfernt das Lösungsmittel im Rotationsverdampfer bei 20 °C und 60 Torr. Anschliessend wird bei 20 °C und 20 Torr der Überschuss an 3,4-Dihydro-2H-pyran abdestilliert.

Es verbleiben 5,8 g gelbes Öl.

Die Kristallisation des Öls setzt in Gegenwart von Impfkristallen und unter Feuchtigkeitsausschluss (Natronkalkrohr) in der Tiefkühltruhe ein. Zur Vervollständigung der Kristallisation werden 4 ml Tetrachlorkohlenstoff (über $K_2CO_3$ getrocknet) gut eingerührt und die Mischung in der Kälte (Kühltruhe) aufbewahrt.

Das abgesaugte Kristallisat wird mit wenig $CCl_4$ gewaschen und im Exsikkator getrocknet (Paraffin/KOH).

Das aus der Mutterlauge gewonnene Öl kristallisiert erst nach Zugabe von 25 ml Petrolether und einer Standzeit von 2 Wochen bei −15 °C voll durch.

Gesamtausbeute: 4,6 g = 92,0% der Theorie.

Herstellung von Acemetacin

Abspaltung der Schutzgruppe des erfindungsgemässen Acemetacinderivates

Der im vorstehenden Versuch hergestellte Tetrahydropyranylester (4,6 g) wird in 30 ml Toluol gelöst, mit 5 mg p-Toluolsulfonsäure versetzt und 1 h bei Raumtemperatur gerührt. Die braune Reaktionslösung wird am Rotationsverdampfer bei 30 °C von Toluol und dem entstandenen 3,4-Dihydro-2H-pyran befreit; erneut zugesetztes Toluol wird auf gleiche Weise entfernt.

Dem in 30 ml Aceton bei 30 °C gelösten Rückstand setzt man 10 ml Wasser und Impfkristalle zu und fügt anschliessend unter Rühren nochmals tropfenweise innerhalb von 1 h 10 ml Wasser zu.

Die Substanz kristallisiert nicht gut durch.

Nach Absaugen und 2maligem Waschen mit Tetrachlorkohlenstoff fällt eine fast farblose Substanz an. Die vom Lösungsmittel befreite Mutterlauge hinterlässt einen Rückstand, der aus Tetrachlorkohlenstoff umkristallisiert weitere 2,9 g liefert.

Gesamtausbeute: 3,9 g = 93,8% der Theorie.

Beispiel 3

Herstellung von 4-Methoxybenzyl-[1-(4-Chlorbenzoyl)-5-methoxy-2-methylindol-3-acetoxy]-acetat

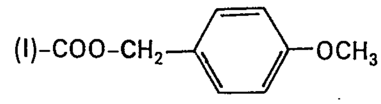

4-Methoxybenzyl-2-bromacetat

20,2 g (0,1 Mol) Bromacetylbromid werden in 100 ml Toluol gelöst, diese Lösung auf −10 °C gekühlt ($CaCl_2$-Rohr) und 8,7 g (0,11 Mol) Pyridin bei −10 °C zugetropft. Anschliessend tropft man zu diesem Gemisch 13,8 g (0,1 Mol) 4-Methoxybenzylalkohol, gelöst in 20 ml Toluol, innerhalb von 20 Minuten bei −10 °C und lässt 1 h bei dieser Temperatur reagieren.

Das auf Raumtemperatur erwärmte Reaktionsgemisch wird 4mal mit Wasser ausgeschüttelt, über $Na_2SO_4$ getrocknet, am Rotationsverdampfer vom Lösungsmittel befreit und bei $10^{-2}$ Torr und 40 °C entgast.

Rückstand: 23,9 g = 92,3 % der Theorie; braunes Öl, das ohne weitere Reinigung im folgenden Syntheseschritt eingesetzt wird.

Acemetacinderivat

7,16 g (0,02 Mol) 1-(4-Chlorbenzoyl)-5-methoxy-2-methyl-indol-3-essigsäure löst man in 40 ml abs. DMF, setzt 1,38 g getrocknetes und pulverisiertes Kaliumcarbonat zu und lässt unter Rühren bei 55 °C die Bildung des Kaliumsalzes ablaufen, bis sich nach 5 h eine klare, gelbe Lösung gebildet hat.

Bei Raumtemperatur fügt man 5,18 g (6,02 Mol) 4-Methoxy-benzyl-2-bromacetat hinzu und lässt 3 h reagieren (pH: 6 bis 7). Das Reaktionsgemisch wird mit 200 ml Essigsäureethylester versetzt, 4mal mit 200 ml Wasser, dann 2mal mit halbkonzentrierter Kaliumbicarbonatlösung ausgeschüttelt und mit Wasser neutral gewaschen. Nach Trocknung der organischen Phase über $Na_2SO_4$, Filtration und Abdampfen des Lösungsmittels am Rotationsverdampfer hinterbleibt ein öliger Rückstand, der aus Ether-Diisopropylether ein farbloses Kristallisat mit einer Ausbeute von 9,4 g = 87,8% der Theorie; Fp.: 88 bis 90 °C, liefert.

Herstellung von Acemetacin

Abspaltung der Schutzgruppe des erfindungsgemässen Acemetacinderivates

Der wie vorstehend beschrieben hergestellte 4-Methoxy-benzylester (5,35 g = 0,01 Mol) wird in 5,4 g (0,05 Mol) Anisol gelöst und unter Rühren und Feuchtigkeitsausschluss mit 0,73 g (0,02 Mol) Eisessig/HCl (75,9 mg HCl/ml) versetzt. Nach 5 h Reaktionszeit ist die Umsetzung quantitativ.

Das Reaktionsgemisch versetzt man mit 1,8 g (0,023 Mol) Ammoniumacetat, rührt 10 Minuten, setzt 20 ml Wasser zu und rührt weitere 10 Minuten. Danach wird mit 40 ml Toluol extrahiert, die organische Phase neutral gewaschen, über $Na_2SO_4$ getrocknet und das Lösungsmittel abdestilliert. Der Rückstand wird mit 200 ml Petrolether ausgerührt. Es entsteht ein Kristallisat, das abgesaugt, mit Petrolether gewaschen und getrocknet wird. Zur Reinigung löst man die Substanz in Methylenchlorid, versetzt mit 80 ml Tetrachlorkohlenstoff und destilliert bei 70 °C das Methylenchlorid ab. Bei Raumtemperatur kristallisiert Acemetacin.

Eine Rekristallisation aus Toluol lieferte Reinsubstanz in einer Ausbeute von 2,8 g = 67,3% der Theorie mit einem Fp.: 150 bis 151 °C.

Beispiel 4

Herstellung von N-(1-Hydroxy-2-methylpropyl-2)-[1-(4-Chlorbenzoyl)-5-methoxy-2-methylindol-3-acetoxy]-acetamid

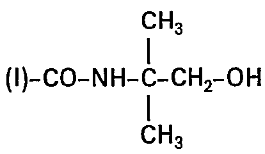

N-(1-Hydroxy-2-methylpropyl-2)-2-bromacetamid

35,6 g (0,4 Mol) 1-Hydroxy-2-methyl-2-aminopropan löst man in 10 ml abs. Methylenchlorid, kühlt auf 0 °C und tropft in diese Lösung unter Feuchtigkeitsausschluss und Einhaltung der Temperatur von 0 °C 40,4 g (0,2 Mol) Bromacetylbromid, gelöst in 100 ml Methylenchlorid, innerhalb von 3 h 50 min ein. 2 h lässt man bei Raumtemperatur nachreagieren. Das 1-Hydroxy-2-methyl-2-aminopropanhydrobromid wird abgesaugt und mit Methylenchlorid gewaschen. Das aus den Filtraten durch Abdampfen des Lösungsmittels erhaltene hellgelbe Öl wird in Ethylenchlorid/Isopropanol (95:5; 9:1; 4:1) über Kieselgel chromatographiert.

Ausbeute: 17,3 g = 41,2% der Theorie; Fp.: 61 bis 63 °C.

Acemetacinderivat

117,9 g (0,05 Mol) 1-(4-Chlorbenzoyl)-5-methoxy-2-methyl-indol-3-essigsäure werden gelöst in 100 ml abs. DMF und nach Zusatz von 3,7 g (0,027 Mol) pulverisiertem Kaliumcarbonat nach 7 h bei 60 °C in das Kaliumsalz überführt. Bei Raumtemperatur setzt man 11,3 g (0,054 Mol) der wie vorstehend beschrieben erhaltenen Verbindung zu und führt die Reaktion 3 h bei 45 °C durch.

Der nach Abdestillieren des Lösungsmittels anfallende ölige Rückstand wird in 200 ml Essigsäureethylester gelöst, durch 3maliges Ausschütteln mit halbkonzentrierter Kaliumbicarbonatlösung von nicht-umgesetztem Ausgangsessigsäurederivat befreit und mit Wasser neutral gewaschen. Die organische Phase hinterlässt nach Trocknung ($Na_2So_4$), Filtration und Verdampfen des Lösungsmittels einen Rückstand, der aus Ether-Disopropylether umkristallisiert wird. Der Rückstand aus der Mutterlauge wird in Cyclohexan-Essigsäureethylester (2:1) über Kieselgel chromatographiert.

Gesamtausbeute: 19,4 g = 79,7 % der Theorie, Fp.: 107–109 °C.

Herstellung von Acemetacin

Spaltung der Schutzgruppe mit Distickstofftetroxid

a) 3,4 g Distickstofftetroxid werden aus einer Bombe unter Feuchtigkeitsausschluss (Siccopentrohr) in einer Kühlfalle mit engem Steigrohr kondensiert und mit kaltem, abs. Tetrahydrofuran auf 12 ml verdünnt (blaue Lösung).

2,4 g (0,005 Mol) des Acemetacinderivates werden in 5 ml Eisessig gelöst, mit 5 ml abs. Dioxan und 1,64 g (0,02 Mol) Natriumacetat versetzt und unter Rühren auf 0 °C gekühlt. Anschliessend tropft man 3,3 ml der Lösung a) bei 0 °C zu und lässt 2 h bei dieser Temperatur reagieren (quantitativer Umsatz des Acemetacinderivates).

Das Reaktionsgemisch giesst man auf Eis und extrahiert mit kaltem Ether. Die Etherphase wird nacheinander gründlich mit eiskaltem Wasser, mit eiskalter Kaliumbicarbonatlösung und Eiswasser gewaschen, über $MgSO_4$ getrocknet, filtriert und am Rotationsverdampfer bei 0 °C auf 80 ml eingeengt. Anschliessend setzt man 0,7 g (0,005 Mol) Kaliumcarbonat (feinpulverisiert) zu und bringt unter Rühren und Feuchtigkeitsausschluss das Gemisch 3 h bei 0 °C zur Reaktion. Die entstandene Gasmenge wird in einer Gasbürette erfasst (bei 0 °C = 165 ml; Theorie: 224 ml). Beim Stehen über Nacht bei Raumtemperatur tritt keine wesentliche Gasentwicklung mehr auf; jedoch ein Substanzniederschlag, aus dem nach Dekantieren der etherischen Lösung, Ansäuern mit Essigsäure und Extraktion mit Essigsäureethylester 700 mg Acemetacin erhalten wurden.

Aus der alkalisch-wässrigen Mutterlauge von obiger Etherextraktion gewinnt man durch Ansäuern und Extraktion weitere 200 mg, hauptsächlich Acemetacin.

In Cyclohexan/Essigsäureethylester/Eisessig (10:10:1) folgt die Chromatographie dieser 900 mg über Kieselgel.

Beispiel 5

Herstellung von (4,4-Dimethyl-2-oxazolin-2-yl)-methyl-1 -(4-chlor- benzoyl)-5-methoxy-2-methylindolacetat

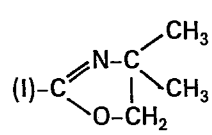

4,4-Dimethyl-2-oxazolin-2-yl-methylbromid

2,1 g (0,01 Mol) 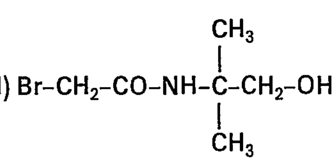

werden unter Feuchtigkeitsausschluss ($CaCl_2$-Rohr) vorgelegt und 3,5 g (0,03 Mol) Thionylchlorid, in 5 ml $CH_2Cl_2$ gelöst, tropfenweise unter Rühren bei Raumtemperatur zugesetzt (Temperaturanstieg auf 26 °C). Anschliessend erwärmt man das Gemisch 2 h auf 40 °C, lässt dann auf Raumtemperatur abkühlen und versetzt unter Rühren mit 25 ml Ether, wobei ein gelbes Öl ausfällt. Dieses wird abgetrennt und nochmals mit 25 ml Ether ausgerührt. Das wiederum isolierte Öl übergiesst man erneut mit 25 ml Ether, kühlt auf −10 °C und setzt unter Rühren 5 ml 20%ige kalte NaOH zu. Nach Abtrennung der Etherphase und erneuter Etherextraktion der wässrigen Phase werden die vereinigten Etherphasen mit NaCl-Lösung ausgeschüttelt, über $Na_2SO_4$ getrocknet, filtriert und am Rotationsverdampfer vom Lösungsmittel befreit.

Rückstand: 0,9 g orangefarbenes Öl, das ohne weitere Reinigung im folgenden Syntheseschritt eingesetzt wird mit folgender Struktur:

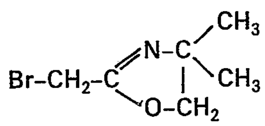

Acemetacinderivat

a) 1,43 g (0,004 Mol) 1-(4-Chlorbenzoyl)-5-methoxy-2-methylindol-3-essigsäure werden in 20 ml abs. DMF gelöst und nach Zusatz von 0,28 g (0,002 Mol) pulverisiertem Kaliumcarbonat unter $N_2$-Schutz, Feuchtigkeitsausschluss und Rühren nach 1,5 h bei 40 °C in das Kaliumsalz überführt. Anschliessend fügt man 0,77 g (0,004 Mol) 4,4-Dimethyl-2-oxazolin-2-yl-methylbromid zu und führt die Reaktion 5,5 h bei 60 bis 70 °C durch (pH: 6 bis 7).

Der nach Abdestillieren von DMF im Wasserstrahlpumpenvakuum bei 40 °C erhaltene Rückstand wird in Essigsäureethylester gelöst, die Lösung 2mal mit Kaliumbicarbonatlösung ausgeschüttelt, getrocknet ($Na_2SO_4$), filtriert und abgedampft.

Rückstand: 1,4 g braunes Öl.

b) Herstellung von

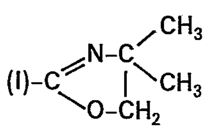 aus 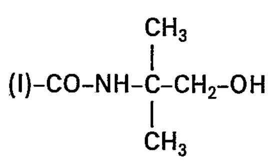

(1) (2)

9,74 g (0,02 Mol) (2) löst man in 20 ml o-Dichlorbenzol und erhitzt unter Rühren, Feuchtigkeitsausschluss ($CaCl_2$-Rohr) und $N_2$-Schutz 5 h zum Rückfluss (185 °C). Das Lösungsmittel wird am Rotationsverdampfer bei 45 °C und $5 \cdot 10^{-2}$ Torr abdestilliert und der hellbraune ölige Rückstand = 9,2 g zur Hochvakuum-Kugelrohrdestillation eingesetzt.

Ausbeute: 6,5 g (1) = 69,3% der Theorie; Kp.: 194 bis 206 °C und $4 \cdot 10^{-4}$ Torr, gelbes Öl, das nach Anreiben und unter Kühlen kristallisiert;

Fp.: 51 bis 53 °C. Das Hydrochlorid hat einen Fp.: 266 bis 270 °C.

Herstellung von Acemetacin

Spaltung der Schutzgruppe in 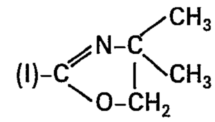

Alle Spaltungsversuche führten primär zu einer Öffnung des Oxazolringes unter Bildung des Zwischenproduktes

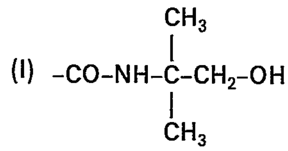

Dieses wird, wie in Beispiel 4 beschrieben, durch Distickstofftetroxid der Amidspaltung in der Seitenkette unter Bildung von Acemetacin unterworfen.

**Patentansprüche**

1. Verfahren zur Herstellung von Acemetacin der Formel

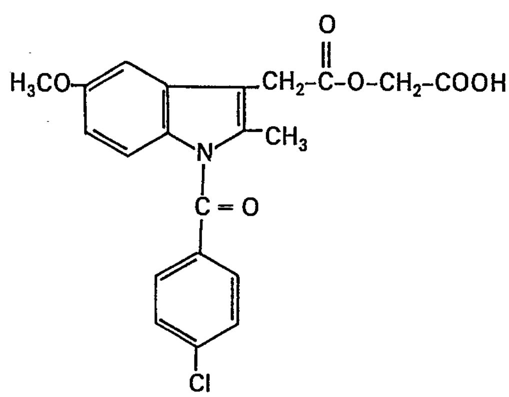

durch Umsetzung von Salzen der Indolcarbosäure der Formel

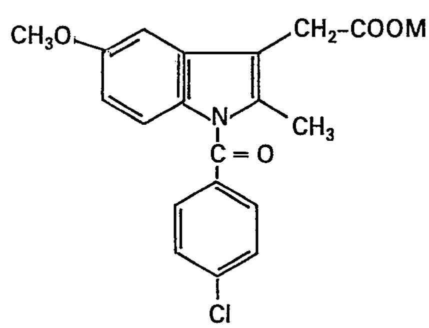

in der

M für Alkali oder der entsprechenden stöchiometrischen Menge Erdalkali steht,

mit Verbindungen der Formel

Hal-$CH_2$-X-R

in der

Hal für Chlor, Brom oder Jod steht,

X für -COO- oder -CONH- und
R für eine Tetrahydrofuran-2-yl, eine Tetrahydropyran-2-yl, eine Alkoxybenzyl- oder eine geradkettige oder verzweigte Alkylgruppe steht, die gegebenenfalls durch eine Hydroxylgruppe substituiert sein kann oder wobei X-R gemeinsam einen Oxazolinring bilden, der durch Alkylgruppen substituiert sein kann, in Gegenwart von inerten organischen Lösungsmitteln oder Gemischen von organischen Lösungsmitteln mit Wasser zu Indolderivaten der Formel

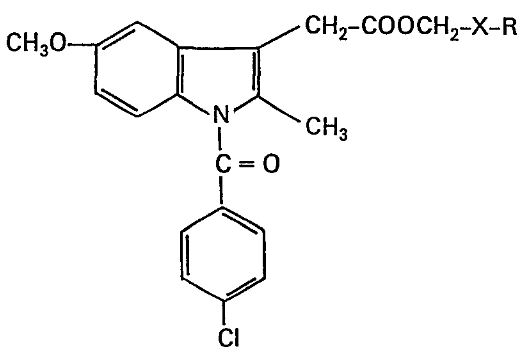

in der
X und R die obengenannte Bedeutung haben, und nachfolgender Hydrolyse, dadurch gekennzeichnet, dass man die Hydrolyse in Gegenwart von Eisessig/Chlorwasserstoff oder p-Toluolsulfonsäure ausführt.

2. Indolderivate der Formel

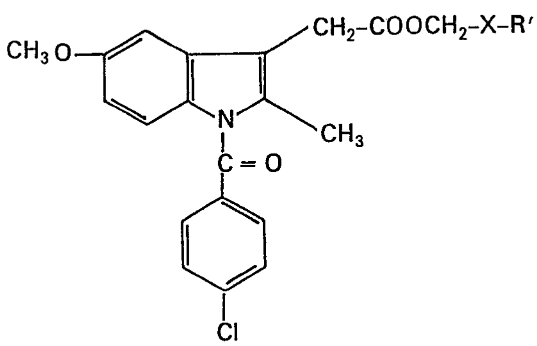

in der
X für -COO- oder -COHN- und
R′ für eine Tetrahydrofuran-2-yl, eine Alkoxybenzyl- oder eine geradkettige oder verzweigte Alkylgruppe steht, die gegebenenfalls durch eine Hydroxylgruppe substituiert sein kann oder wobei X-R gemeinsam einen Oxazolinring bilden, der durch Alkylgruppen substituiert sein kann, sowie ihre mit Basen gebildeten pharmazeutisch unbedenklichen Salze.

3. Verfahren zur Herstellung von Indolderivaten nach Anspruch 2, dadurch gekennzeichnet, dass man Salze der Indolcarbonsäure der Formel

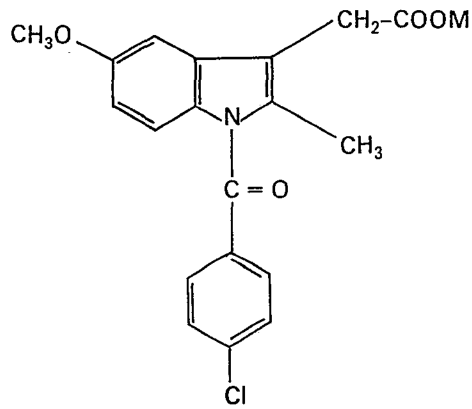

in der
M für Alkali- oder entsprechende stöchiometrische Mengen von Erdalkalimetallen steht, mit Verbindungen der Formel

$Hal-CH_2-X-R'$

in der
Hal für Chlor, Brom oder Jod steht und
X und R′ die in Anspruch 2 angegebene Bedeutung besitzen, in Gegenwart von inerten organischen Lösungsmitteln oder Gemischen von organischen Lösungsmitteln mit Wasser in einem Temperaturbereich von −30 bis +70 °C umsetzt.

**Claims**

1. Process for the preparation of acemetacin of the formula

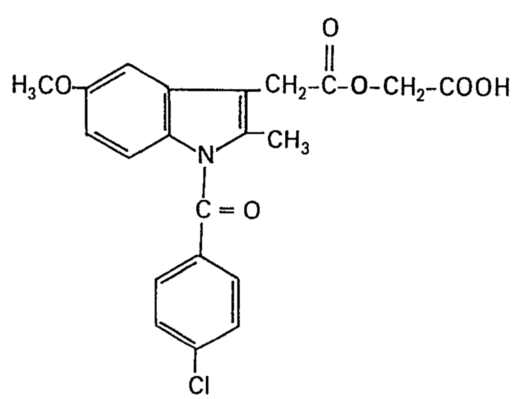

by reacting salts of the indolecarboxylic acid of the formula

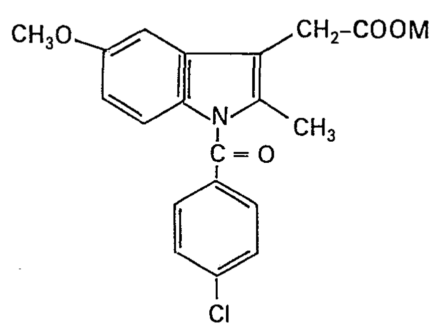

in which
M represents an alkali metal or the corresponding stoichiometric quantity of an alkaline earth metal,
with compounds of the formula

$Hal-CH_2-X-R$

in which
Hal represents chlorine, bromine or iodine,
X represents -COO- or -CONH- and
R represents a tetrahydrofuran-2-yl, a tetrahydropyran-2-yl, an alkoxybenzyl or a straight-chain or branched alkyl group which can optionally be substituted by a hydroxyl group, or wherein X-R together form an oxazoline ring which can be substituted by alkyl groups, in the presence of inert organic solvents or mixtures of organic solvents with water, to form indole derivatives of the formula

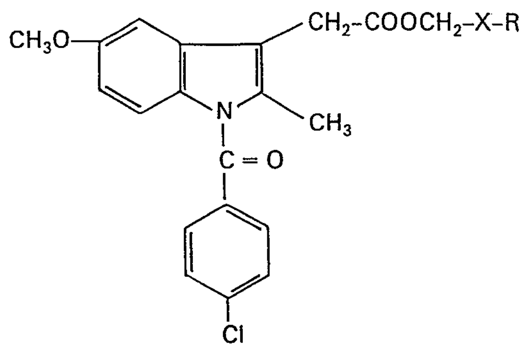

in which
X and R have the abovementioned meaning, followed by hydrolysis, characterised in that the hydrolysis is carried out in the presence of glacial acetic acid/hydrogen chloride or p-toluenesulphonic acid.

2. Indole derivatives of the formula

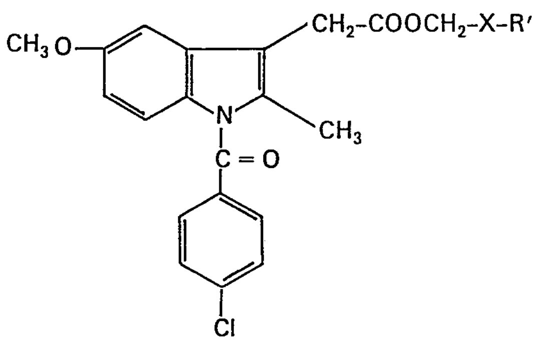

in which
X represents -COO- or -COHN- and
R' represents a tetrahydrofuran-2-yl, an alkoxybenzyl or a straight-chain or branched alkyl group which can optionally be substituted by a hydroxyl group, or wherein X-R together form an oxazoline ring which can be substituted by alkyl groups and their pharmaceutically acceptable salts formed with bases.

3. Process for the preparation of indole derivatives according to Claim 2, characterised in that salts of the indolecarboxylic acid of the formula

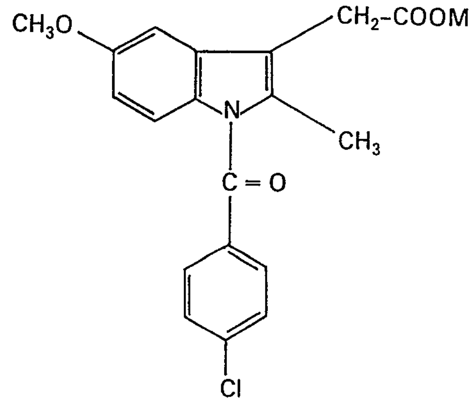

in which
M represents alkali metals or corresponding stoichiometric amounts of alkaline earth metals, are reacted with compounds of the formula

$Hal-CH_2-X-R'$

in which
Hal represents chlorine, bromine or iodine and
X and R' have the meaning given in Claim 2, in the presence of inert organic solvents or mixtures of organic solvents with water in a temperature range from −30 to +70 °C.

**Revendications**

1. Procédé de préparation de l'Acemetacin de formule

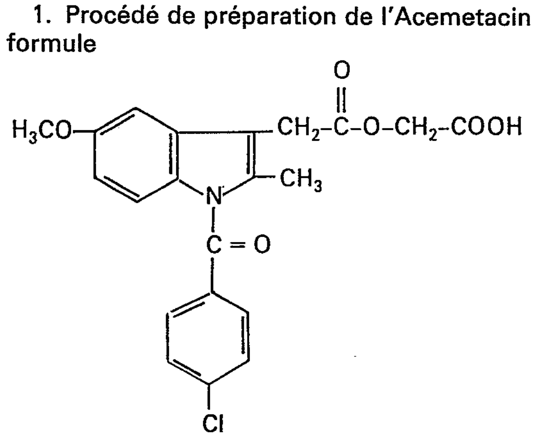

par réaction de sels de l'acide indole-carboxylique de formule

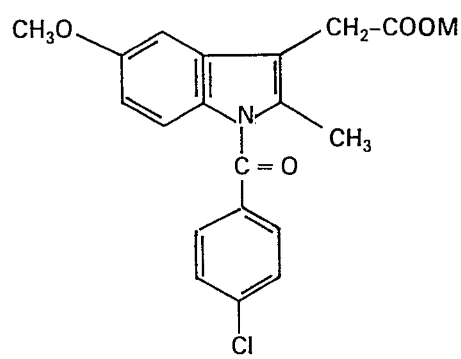

dans laquelle
M représente un métal alcalin ou la quantité théorique correspondante de métal alcalino-terreux,
avec des composés de formule

$Hal-CH_2-X-R$

dans laquelle
Hal représente le chlore, le brome ou l'iode,
X représente -COO- ou -CONH- et
R représente un groupe tétrahydrofuranne-2-yle, tétrahydropyranne-2-yle, alcoxybenzyle ou un groupe alkyle droit ou ramifié, qui peut le cas échéant être substitué par un groupe hydroxy, ou bien X-R, ensemble, forment un cycle oxazoline qui peut être substitué par des groupes alkyle, en présence de solvants organiques ou de mélanges de solvants organiques inertes avec de l'eau, avec formation de dérivés de l'indole de formule

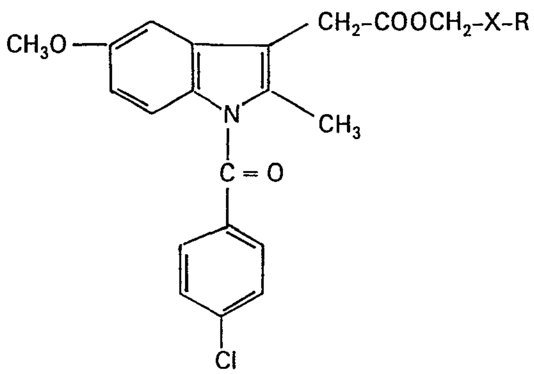

dans laquelle
X et R ont les significations indiquées ci-dessus, et hydrolyse subséquente, caractérisé en ce que l'on effectue l'hydrolyse en présence d'un mélange acide acétique glacial/acide chlorhydrique ou d'acide p-toluène-sulfonique.

2. Dérivé de l'indole de formule

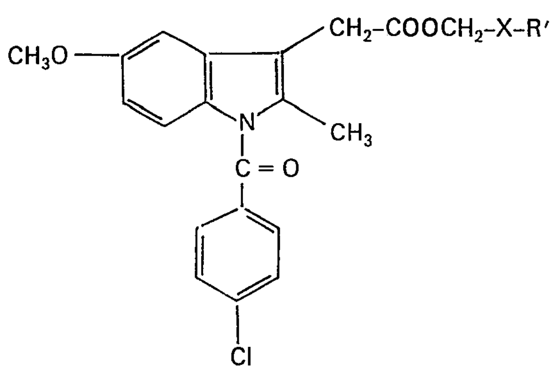

dans laquelle
X représente -COO- ou -COHN- et
R' représente un groupe tétrahydrofuranne-2-yle, un groupe alcoxy-benzyle ou un groupe alkyle à chaîne droite ou ramifiée, qui peut le cas échéant être substitué par un groupe hydroxy, ou bien X-R ensemble forment un cycle oxazoline qui peut être substitué par des groupes alkyle, et leurs sels acceptables pour l'usage pharmaceutique formés avec des bases.

3. Procédé de préparation des dérivés de l'indole selon la revendication 2, caractérisé en ce que l'on fait réagir les sels de l'acide indole-carboxylique de formule

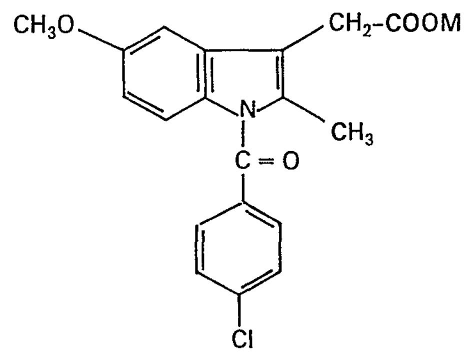

dans laquelle
M représente un métal alcalin ou la quantité théorique correspondante d'un métal alcalino-terreux,
avec de composés de formule

$Hal-CH_2-X-R'$

dans laquelle
Hal représente le chlore, le brome ou l'iode et
X et R' ont les significations indiquées dans la revendication 2, en présence de solvants organiques inertes ou de mélanges de solvants organiques avec de l'eau, dans un intervalle de températures de −30 à +70 °C.